# EUROPÄISCHE PATENTSCHRIFT

(11) **EP 1 374 915 B1**
(45) Veröffentlichungstag und Bekanntmachung des Hinweises auf die Patenterteilung: **27.04.2011**
(21) Anmeldenummer: 03012768.2
(22) Anmeldetag: 05.06.2003
(51) Int. Cl.: A61L 2/08, A61K 6/10

(54) **Verfahren zur Keimreduktion von Abformmaterialien**
Method for germ reduction of impression materials
Procédé de réduction de germes des matériaux d'empreinte dentaires ou médicales

(30) Priorität: 25.06.2002 DE 10228421
(43) Veröffentlichungstag der Anmeldung: 02.01.2004
(73) Patentinhaber: Heraeus Kulzer GmbH, 63450 Hanau (DE)
(72) Erfinder: Grunwald, Martin, Dr., 50259 Pulheim (DE); Esser, Birgit, 41515 Grevenbroich (DE); Weber, Norbert, 50769 Köln (DE)
(74) Vertreter: Kühn, Hans-Christian

(56) Entgegenhaltungen:
- EP-A- 0 164 190
- EP-A- 0 265 776
- WO-A-00/07546
- DE-A- 19 719 438
- US-A- 5 540 876
- DATABASE WPI Derwent Publications Ltd., London, GB; AN 1975-26486W XP002252986 & JP 52 013234 B (T HIRASAWA), 13. April 1977 (1977-04-13)

## Beschreibung

Die vorliegende Erfindung betrifft ein Verfahren zur Keimreduktion von Abformmaterialien.

Unterschiedliche Arten an Abformmassen sind an sich bekannt (siehe R.G. Craig, Restaurative Dental Materials, The C.V. Moosbe-Comp. St. Louis, Toronto, London, 1980, S. 1979 ff). An derartige Materialien werden insgesamt sehr hohe Anforderungen gestellt (vergleiche K. Eichner, Zahnärztliche Werkstoffe und ihre Verarbeitung, Bd. 1, A. Hüthig Verlag, Heidelberg, 4. Auflage, 1981, S. 45 ff):
1. Angenehmer Geruch, Geschmack und ästhetisches Aussehen.
2. Die Massen dürfen keine toxischen oder irritierenden Bestandteile enthalten.
3. Die Massen müssen eine mehrmonatige Lagerstabilität aufweisen.
4. Die Massen müssen wirtschaftlich herstellbar sein und eine präzise Abformung ergeben.
5. Die Massen müssen leicht zu handhaben sein.
6. Die Härtungscharakteristik muss den klinischen Erfordernissen entsprechen.
7. Die ausgehärteten Massen müssen elastisch sein und dürfen sich nicht unter Zugbeanspruchung bleibend verformen.
8. Die ausgehärteten Massen müssen eine ausreichende Druckfestigkeit besitzen und dürfen nicht brechen.
9. Die ausgehärteten Massen müssen bei Raumtemperatur und normaler Luftfeuchtigkeit solange dimensionsstabil sein, dass in angemessener Zeit exakte Gipsabdrücke hergestellt werden können.
10. Die ausgehärteten Massen dürfen keine Gipsschädigung hervorrufen und müssen mit anderen Abformmassen kompatibel sein.

Aus der Gruppe der verschiedenen Materialien sind elastomere Abformmaterialien besonders vorteilhaft, unter anderem auch auf Grund der gegenüber den nicht elastomeren Abformmaterialien vorteilhaften anwendungstechnischen und mechanischen Eigenschaften.

Es sind verschiedene Arten von elastomeren Abformmaterialien bekannt, wie zum Beispiel Elastomere mit Polymerkettenstruktur, die durch Additionsreaktion abbinden (wie zum Beispiel additionsvernetzende Silicon-Abformmaterialien (sogenannte A-Silicone), die durch eine Hydrosilierungsreaktion von Vinylgruppen an einem polydiorganylgruppenhaltiges Polymer (Vinylpolymere) mit einem SiH-Gruppen enthaltenden Polydiorganosiloxan (SiH-Vernetzer) miteinander reagieren und dadurch ein Elastomer formen, beziehungsweise entsprechende Polyethermaterialien (wie sie zum Beispiel in DE-A1-3741575 beziehungsweise DE-A1-3838587 beschrieben sind)), oder aber durch Kondensationsreaktion Elastomere bildende Abformmassen, wie zum Beispiel kondensationsvernetzende Silicon-Abformmate-rialien (sogenannte C-Silicone) oder aber Polyether-Abformmaterialien (wie sie zum Beispiel in DE 101 04 079.2-42 und zum Beispiel in der dort gewürdigten EP 0 269 819 B1 beschrieben werden). Andere häufig verwendete elastomere Abformmaterialien sind solche mit Polyetherketten und Verknüpfung über Aziridinogruppen (wie zum Beispiel in DE-B-17 45 810 beschrieben); ebenso sind Polyetherabformmaterialien mit Acrylat- beziehungsweise Meth-acrylatgruppen, zum Beispiel aus EP 0 173 085, bekannt. Diese Abformmaterialtypen erfüllen im Wesentlichen die zuvor genannten generellen Eigenschaften für Abformmaterialien.

In der Regel liegen diese elastomeren Abformmaterialien vor der "Abbindung" (das heißt, Formung der elastomeren Struktur) als Pasten vor, die in der Regel aus zwei Komponenten (häufig als Basispaste und Katalysator- oder Härterpaste bezeichnet) bestehen und zu dem Elastomer nach Vermischen abbinden (vernetzen).

Durch ein Abformmaterial wird in den zuvor genannten Anwendungsbereichen der Negativabdruck einer Situation der körpereignen Geometrie angefertigt, zum Beispiel zur Herstellung eines Ersatzteils oder aus diagnostischen Gründen, oder aber ein Stempelmaterial erzeugt. Dabei soll die Situation möglichst detailgenau wiedergegeben werden. Das heißt, aus diesem Grund empfehlen sich insbesondere elastomere Abformmaterialien, die nicht nur eine hohe Detailgenauigkeit und Dimensionsbeständigkeit auch bei Lagerung der Abformung aufweisen, sondern sich auch ohne wesentliche Änderung der physikalischen Eigenschaften gut desinfizieren lassen, was bei Verwendung in dem medizinischen Bereich insofern von besonderer Bedeutung ist, da die abgeformten Körperpartien eine mehr oder minder intensive Keimbesiedelung aufweisen, die zu einer Kontamination mit diesen Keimen bei nachfolgenden Arbeitsschritten, wie Modellherstellung, Erstellung der restaurativen Arbeit etc., führen und letztlich auch die mit diesen Abformungen und den mit Hilfe dieser Abformungen daraus hergestellten Ersatzteilen beschäftigten Personen erheblich gefährden können. Daher existieren viele Verfahren zur Desinfektion von ausgehärteten Abformungen (zum Beispiel mittels H₂O₂, UV-Bestrahlung, Anwendung von Desinfektionsmitteln (zum Beispiel in F.M. Blair, R.W. Wassell, British Dental Journal, Vol. 180, No. 10, 1996, S. 369 ff. beziehungsweise G.L. Adabo, E. Zanarotti, R.G. Fonseca, C.A. Cruz, Journal of Prosthetic Dentistry 81 (5), 1999, S. 621 ff.) oder γ-Strahlensterilisation von Abformungen (zum Beispiel in J. Setz, U. Benzing, Deutsche Zahnärtzliche Zeitschrift, 44, 1989, S. 106 f.)). Alle diese Verfahren werden jedoch an den bereits "abgebundenen", das heißt, elastomeren Abformungen durchgeführt.

In den zuvor genannten medizinischen Anwendungsbereichen tritt jedoch häufig bei der Abdrucknahme das Problem auf, dass das Abformmaterial im nicht abgebundenen beziehungsweise nicht vernetzten Zustand mit verletztem Haut- oder Schleimhaut- oder Knochengewebe in Verbindung kommt (zum Beispiel durch Blutungen an der Schleimhaut bei der dentalen Abdrucknahme beziehungsweise bei Abformungen beim Setzen von Implantaten oder aber bei Abformungen bei noch nicht verheiltem Hautgewebe bei der Abformung im Rahmen von epithetischen Versorgungen oder bei Hautabformungen).

Dabei besteht naturgemäß die Gefahr, dass diese mit dem Abformmaterial in Berührung kommenden Körperpartien mit Keimen (wie zum Beispiel Bakterien, Bazillen, Pilze, Hefen, Viren) aus dem Abformmaterial beziehungsweise aus dem Primärpackmittel beziehungsweise von dem zur Applikation benötigten Zubehör (wie zum Beispiel Mischkanülen für Abformmaterialien, die in Doppelkammerkartuschen angeboten werden, oder aber Anmischspatel) kontaminiert werden; diese Keimkontamination kann zu schwerwiegenden gesundheitlichen Problemen führen, die bei Personen mit eingeschränkter Funktion des Immunsystems besonders negative Auswirkungen haben können.

Darüber hinaus ist es bei den sogenannten Stempeltechniken (wie sie unter anderem in den Grundzügen beschrieben werden bei Y. Xia, G.M. Whitesides, Angew. Chem. Int. Ed. 1998, 37, 550 - 575; E. Delamarche, H. Schmid, B. Michel, H. Biebuyck, Adv. Mater. 1997, 9, 741 - 746; H. Schmid, B. Michel, Macromolecules 2000, 33, 3042 - 3049), bei denen Substrate oder Strukturen mittels eines Stempels auf Oberflächen übertragen werden, wünschenswert, dass diese Stempelmaterialien keimfrei sind, insbesondere dann, wenn biologische oder arzneilich wirksame Materialien durch diese Technik übertragen werden. Hier verbietet sich naturgemäß die nachträgliche Desinfektion des (mehrfach benutzbaren) Stempels (da dadurch Desinfektionsmittelreste in das biologische Substrat übertragen werden und die biologische Wirkung beeinflussen können) ebenso wie die Beimengung von antimikrobiell wirksamen Substanzen in das Stempelmaterial (die ja ebenfalls in das biologische Substrat übertragen werden können oder aber eine oberflächliche Inaktivierung des biologischen Substrates bewirken können).

Es hat daher nicht an Versuchen gefehlt, Maßnahmen zu treffen, die diese Gefahr der Verkeimung beseitigen.

So wurde von D.N. Firtell, D.J. Moore, G.B. Pelleu Jr. in Journal of Prosthetic Dentistry, 1972, S. 419 - 422 ein Verfahren beschrieben, um Alginatpulver mit Ethylenoxidbegasung zu sterilisieren. Diese für pulverförmige Materialien geeignete Methode lässt sich jedoch nicht in einfacher Weise auf Abformmaterialpasten übertragen.

Ebenso wurde versucht, einen keimarmen oder sogar sterilen Abdruck durch Zusatz keimtötender Mittel zu den Abformmaterialien zu erzeugen, indem das Keimwachstum verhindernde Mittel den Materialien zugegeben wurden (zum Beispiel DE 37 24 243, JP 07112910, WO 99/15132, WO 00/07546). Diese Art der Problemlösung birgt die Gefahr einer Eigenschaftsveränderung des so ausgestatteten Abformmaterials, und vor allen Dingen haben alle entsprechenden Zusätze den Nachteil, dass dadurch auch die entsprechende Körperpartie kontaminiert wird mit allen damit verbundenen Nachteilen, wie zum Beispiel lokale Reizungen bis hin zu möglichen grundsätzlichen Unverträglichkeiten beziehungsweise allergischen Reaktionen durch die wirksamen Bestandteile. Darüber hinaus haben antimikrobiell wirksame Wirkstoffe in der Regel ein eingeschränktes Wirkspektrum auf spezielle Keime. Wie bereits erläutert, verbietet sich eine solche Vorgehensweise bei der Anwendung der sogenannten Stempeltechnik.

In US-Patent 4,033,774 wird ein thermoplastisches, nicht elastomeres dentales Abformmaterial beschrieben, das vor Gebrauch in einem Autoklaven sterilisiert werden können soll, ohne dass dazu weitere Details angegeben werden. Eine generell anwendbare Problemlösung für eine mögliche Keimreduktion bei elastomeren Abformmaterialien wird dadurch nicht beschrieben. Von Th. Kaus, A. Sethi wird in ZWR, 110. Jahrgang, 2001, S. 22- 26 die Verwendung eines strahlensterilisierten Abformlöffels und einer strahlensterilisierten Mischkanüle für ein Abformmaterial in einem Kartuschensystem beschrieben, nicht aber die Verwendung eines sterilisierten Abformmaterials.

Ein Beispiel für γ-Strahlenbehandlungen von im Dentalbereich anwendbaren Kunststoffen ist durch JP 52013234 B4 gegeben, wobei eine gravierende Elastizitätsveränderung eines im Dentalbereich einsetzbaren Kunststoffs durch y-Bestrahlen von Polymethylmethacrylatpulver beschrieben wird. Dieses beschriebenen Material ist nicht als Abformmaterial nutzbar, und auf eine Keimreduktion wird nicht eingegangen.

US-Patent 5,540,876 beschreibt ein thermoplastisches, nicht elastomeres γ-strahlenbehandeltes Poly(epsilon-Caprolacton) und dessen Eigenschaftsänderungen durch γ-Strahlenbehandlung, die das Material für die Verwendung in der Dentaltechnik gebräuchlich macht; das Material ist als elastomeres Abformmaterial ebenfalls nicht nutzbar.

Es muss also festgestellt werden, dass nach dem gegenwärtigen Stand der Technik kein allgemein anwendbares Verfahren zur Erzeugung einer keimreduzierten oder sterilen Abformmasse oder Masse zur Erstellung eines Stempels, die in dieser keimarmen Form vor der Abdrucknahme beziehungsweise vor der Erstellung des Stempels vorliegt, bekannt ist.

Der Erfindung liegt die Aufgabe zugrunde, ein Verfahren zur sicheren Keimreduktion von Abformmaterialien anzugeben.

Die Aufgabe wird erfindungsgemäß durch die Verfahren gemäß den Ansprüchen 1 bzw. 2 gelöst, dem die Komponenten der Abformmaterialien einer Strahlensterilisation unterworfen werden. Dabei werden zweikomponentige, zu einem elastomeren Material vernetzte Abformmaterialien verwendet. Ferner ist es erfindungsgemäß, additions-, kondensations- oder über (Meth)acrylatgruppen vernetzbare Silicon-Abformmassen bzw. additions-, kondensations-, über Ringöffnung oder über (Meth)acrylatgruppen vernetzbare Polyetherabformmassen zu verwenden. Weiterhin ist es erfindungsgemaß ein additionsvernetzendes Silicon-Abformmaterial zu verwenden, das in der Formulierung vinylgruppenhaltige Polysiloxane mit zumindest teilweise vorhandenen Diphenylsiloxan- und/oder Phenylmethylsiloxan-Struktureinheiten aufweist. Bevorzugt werden auch Polymere verwendet, die mindestens 3 Mol-%, vorzugsweise mindestens 10 Mol-% Diphenylsiloxan und/oder Phenylmethylsiloxan-Einheiten aufweisen.

Erfindungsgemäß wird das Verfahren verwendet im medizinischen Bereich, insbesondere im Dentalbereich, in der Otoplastik, Orthopädie, Epithetik, Defektchirurgie, im Bereich der Abformungen in der HNO-Heilkunde, der Veterinärmedizin oder der Abformung von Hautpartien, sowie als Stempelmaterial für Stempeltechniken, insbesondere bei Verwendung von biologischen oder arzneilich wirksamen Substraten. Es konnte festgestellt werden, dass Abformmassen, insbesondere zur Verwendung in dem zuvor genannten medizinischen Bereich und/oder als Stempelmaterial in den sogenannten Stempeltechniken, insbesondere vor der Vernetzung durch energiereiche Strahlung, bevorzugt durch Gammastrahlung beziehungsweise Elektronenstrahlung (β-Strahlung), keimreduziert beziehungsweise sterilisiert werden können. Unter dem Begriff Strahlensterilisation soll sowohl eine Keimreduktion als auch eine Sterilisation verstanden werden.

Das ist angesichts der zum Beispiel in JP 52013234 B4 und US-Patent 5,540,876 beschriebenen Eigenschaftsveränderungen (Strahlenvernetzung, Abbau von Polymerstrukturen) nicht so zu erwarten gewesen. Für hohe Strahlendosen sind Ab- und Umbaumechanismen von in den zuvor genannten Abformmassen zum Teil benutzten Polydimethylsiloxanketten bekannt (D.J.T. Hill, C.M.L. Preston, A.K. Whittaker, S.M. Hunt, Macromol. Sym. 156, 95 - 102 (2000)). Vor dem Hintergrund einer möglichen Strahlenvernetzung, wie sie zum Beispiel in W. Noll, Chemie und Technologie der Silicone, Verlag Chemie, Weinheim, 2. Auflage, 1968, S. 199 f., beschrieben werden, ist eine solche Beständigkeit entsprechender Silicon-Abformmassen nicht zu erwarten gewesen.

Sterilisationen durch Bestrahlungen sind grundsätzlich bekannt (zum Beispiel in K.H. Wallhäuser, Praxis der Sterilisation - Desinfektion - Konservierung - Keimidentifizierung - Betriebshygiene, 3. Auflage, Georg Thieme Verlag, Stuttgart - New York, 1984, Kap. 3.4), ohne dass Abformmassen für die vorgenannten Zwecke entsprechend sterilisiert wurden.

Die Prinzipien der γ- und β-Strahlensterilisation sind in der oben genannten Monographie von K.H. Wallhäuser oder auch anderen Monographien ausführlich beschrieben. Elektronenstrahlsterilisation wird mit Hilfe von Elektronenbeschleunigern durchgeführt, die die beschleunigten Elektronen auf das zu bestrahlende Material schießen und so innerhalb kurzer Zeit eine große Dosis einwirken lassen. Nachteilig im Vergleich zu der daher als besonders bevorzugt anzusehenden Sterilisation durch γ-Strahlen ist das deutlich geringere Durchdringungsvermögen der β-Strahlen, so dass die Bestrahlung mit β-Strahlen wirtschaftliche Nachteile gegenüber der γ-Bestrahlung durch die Probleme, die vorgenannten Abform- beziehungsweise Stempelmassen im Bulk zu sterilisieren (und der damit verbundenen Notwendigkeit, die Verpackungen mit dem Material in nur dünnen Schichtstärken zu sterilisieren), mit sich bringt.

Das Durchdringungsvermögen bei Gammastrahlenbehandlung ist ungleich größer. Bei der Gammastrahlenbehandlung wird das zu sterilisierende Gut an einer Strahlenquelle, in der Regel ein gekapseltes Isotop ⁶⁰Co, vorbeigeführt, bis die gewünschte Dosis erreicht ist. Das größere Penetrationsvermögen der γ-Strahlensterilisation führt dazu, dass die zu sterilisierenden Massen in größeren Einheiten sterilisiert werden können.

Die Behandlungstechniken mit γ- beziehungsweise β-Strahlen sind an sich bekannt.

Je nach Strahlendosis können unterschiedliche Keimreduktionsraten erzielt werden, die jedoch auch von den als Keimbesiedlung vorliegenden Keimen abhängig sind. Häufig wird bei Strahlensterilisationen von Medizinprodukten mit 25 kGy gearbeitet (vergleiche zuvor genannte Monographie von K.H. Wallhäuser, S. 233). Insbesondere bei den Abformmassen beziehungsweise Materialien für die Stempeltechnik kann, da die Ausgangskeimbelastung durch betriebliche Hygienemaßnahmen bei der Herstellung als gering anzusehen ist (häufig mittlerer Bioburden von 5 - 10), die Strahlendosis eventuell noch weiter abgesenkt werden, um noch hinreichende Wahrscheinlichkeit für "Sterilität" (das heißt, Wahrscheinlichkeit, ein verkeimtes Produkt zu finden, liegt bei kleiner 1 : 1 Million) gewährleistet zu haben (Hinweise dazu sind aus Tabelle B1 der ISO 11137: 1995 (E) zu entnehmen), so dass 20 kGy als Strahlendosis häufig ausreichend sein dürften. Als Strahlendosis werden je nach verwendetem Abformmaterial höchstens 50 kGy, bzw. höchstens 30 kGy, vorzugsweise 20 bis 30 kGy, verwendet.

Geringe Strahlendosen, die noch keine sichere Sterilität gewährleisten, können zumindest eine erhebliche Keimreduktion bewirken.

Je nach Bioburden und je nach Zielsetzung (Keimreduktion, Sterilität) kann die Strahlendosis festgelegt werden.

Grundsätzlich können alle Arten von Abformmassen, die in den genannten medizinischen Bereichen beziehungsweise als Stempelmaterial durch Bestrahlung keimreduziert beziehungsweise sterilisiert werden. Erfindungsgemäß sind die vorstehend beschriebenen, nach Vernetzung elastomeren Abformmassen auf Basis vernetzbarer Polyether- beziehungsweise Polysiloxanpolymergerüste.

Wichtig in diesem Zusammenhang ist es, dass beim bestrahlten Material nur geringe Veränderungen der physikalischen beziehungsweise anwendungstechnischen Eigenschaften gegenüber dem unbehandelten Material auftreten.

Überraschenderweise wurde gefunden, dass Polyether-Abformmaterialien und kondensationsvernetzende Siliconabformmaterialien auch bei vergleichsweise hohen Strahlendosen von zum Beispiel 40 kGy keine anwendungswesentliche Veränderung zeigen. Interessanterweise sind additionsvernetzende Silicon-Abformmassen mit Polydimethylsiloxan-polymer-gerüst bei höheren Strahlendosen von zum Beispiel 40 kGy empfindlicher; es tritt hier eine Vernetzung (Strahlenvernetzung der Vinylgruppen) auf, die die Gebrauchsfähigkeit einschränkt. Auch bei geringeren Strahlendosen zeigen sich bei additionsvernetzenden Silicon-Abformmaterialien Vorvernetzungen, die sich folgerichtig auch in schnelleren Vernetzungskinetiken zeigen. Das heißt, bei üblichen additionsvernetzenden Silicon-Abformmaterialien auf Basis von Polydimethylsiloxanpolymeren ist es zwar möglich, diese bei geeigneten Strahlendosen und noch akzeptablen anwendungstechnischen Eigenschaften zu sterilisieren, die so erhaltenen Materialien zeigen jedoch deutliche Veränderungen gegenüber den Ausgangsmaterialien.

Aus G.G. Delidest, Radiat. Phys. Chem., 16, (1980), S. 345- 352 ist eine "protektive" Wirkung von Phenylgruppen auf das Verhalten von bestrahlten Dimethyldiphenylsiloxanen bekannt. Ähnliche Aussagen finden sich auch in W. Noll, Chemie und Technologie der Silicone, Verlag Chemie, Weinheim, 2. Auflage, 1968, S. 407 f. und 447.

Interessanterweise wurde gefunden, dass der Einsatz von phenylgruppenhaltigen und vinylgruppenhaltigen Polydimethylsiloxanpolymeren in additionsvernetzenden Silicon-Abformmassen zu signifikant geringeren Veränderungen führt, so dass es bevorzugt ist, Siloxanpolymere mit mindestens 3 Mol-%, besonders bevorzugt mindestens 10 Mol-%, an Diphenylsiloxan- und/oder Methylphenylsiloxaneinheiten einzusetzen.

Das heißt, für jeden Abformmaterialtyp muss die sich aus physikalischen beziehungsweise anwendungstechnischen Eigenschaftsveränderungen heraus ergebende maximale Strahlendosis unter Berücksichtigung der Ausgangsverkeimung und des angestrebten Grades der Keimreduktion festgelegt werden. Für die üblichen zu einem Elastomer vernetzenden zweikomponentigen Abform- beziehungsweise Stempelmassen auf Basis von Polyether- beziehungsweise Kondensationsvernetzbaren oder über (Meth)acrylatgruppen vernetzbaren Siliconpolymeren sind maximale Strahlendosen von 40 kGy bevorzugt, besonders bevorzugt maximale Strahlendosen von 30 kGy.

Erfindungsgemäß ist es, diese Abform- beziehungsweise Stempelmassen im Primärpackmittel zu sterilisieren, besonders vorteilhaft ist es, auch das zum Mischen und Applizieren notwendige Zubehör mit zu sterilisieren (wobei es natürlich auch durchaus möglich ist, dass das Primärpackmittel von außen beziehungsweise auch das Zubehör (zum Beispiel Mischspatel, Mischdüsen etc.) durch eine andere Methode keimreduziert oder sterilisiert wird (zum Beispiel Sterilisation durch Ethylenoxid, Niedertemperaturplasmasterilisation mit H₂O₂ oder ähnliches)).

Vorteilhaft ist eine Sterilisation durch γ- oder β-Strahlung von Material im Primärpackmittel, gegebenenfalls zusammen mit notwendigem Zubehör in einer dichten Endverpackung (zum Beispiel verschweißter Beutel); besonders vorteilhaft ist eine Doppelkammerkartusche als Primärpackmittel und Mischdüsen (Mixing Tips) als Zubehör. Ganz besonders vorteilhaft ist es, die Abform- beziehungsweise Stempelmasse im Primärpackmittel nebst Zubehör in einer für eine einmalige Applikation ausreichenden Menge keimreduziert beziehungsweise steril zur Verfügung zu stellen.

Dabei ist natürlich darauf zu achten, dass die verwendeten Packmittel, das Zubehör und die Folien- beziehungsweise Umverpackungsmaterialien die Sterilisationsbedingungen durch energiereiche Strahlung vertragen. Solche strahlensterilisationsstabile Verpackungen beziehungsweise Zubehör sind an sich bekannt

Beim der Auswahl von Primärpackmittel und Zubehör ist natürlich zusätzlich darauf zu achten, dass die verwendeten Materialien gegenüber den entsprechenden Abformmaterialtypen gute Beständigkeit zeigen.

Die nachfolgenden Beispiele sollen die vorliegende Erfindung beschreiben, ohne sie einzuschränken.

### Beispiel 1:

Bei diesen Versuchen wurden verschiedene Abformmassetypen (Silicon-Abformmasse (kondensationsvernetzend), Polyetherabformmassen (kondensationsvernetzend beziehungsweise ringöffnend vernetzend)) in Doppelkammerkartuschen mit Gammastrahlen von 25 kGy bestrahlt und danach mit den unbehandelten Proben vergleichend untersucht. Die Ergebnisse sind in der nachfolgenden Tabelle zusammengestellt und zeigen gute Stabilität der untersuchten Abformmassetypen. Alle auf die Strahlenbehandlung zurückzuführenden Veränderungen in der Kinetik und in den Viskositäten sind in einem auch anwendungstechnisch durchaus akzeptablen Bereich.

### Ergebnisse der Vergleichsversuche von unsterilisierten gegen sterilisierte Proben bei einer γ-Bestrahlung von 25 kGy

| **Material** | | **Aziridinopolyether** | | **Kondensationsvernetzendes Polyether-Abformmaterial** | | **Silicon-Abformmaterial (kondensationsvernetzend)** | |
|---|---|---|---|---|---|---|---|
| | | Impregum Garant L Duo Soft (2:1) | | URH 0234-1/URH 0234-2 (4:1) | | Xantopren Comfort Medium (4:1) | |
| | | unbehandelt | bestrahlt | unbehandelt | bestrahlt | unbehandelt | bestrahlt |
| Viskosität Basis | [Pas] | 84 | 86 | 85 | 71 | 24 | 26 |
| Viskosität Katalysator[Pas] | | 249 | 220 | 90 | 76 | 3 | 3 |
| Verarbeitungszeit nach OSC-Methode | [min] | 2,45 | 2,40 | 1,65 | 2,30 | 2,60 | 2,25 |
| Abbindezeit nach OSC-Methode | [min] | 3,50 | 3,50 | 2,75 | 3,65 | 3,85 | 3,55 |
| Rückstellung nach Verformung | [%] | 97,7 | 97,7 | 98,4 | 98,2 | 97,5 | 97,8 |

### Beispiel 2:

Es wurden unterschiedliche Abformmassetypen einer γ-Strahlenbehandlung bei unterschiedlichen γ-Strahlendosen ausgesetzt und die bestrahlten und nicht bestrahlten ("O-Proben") Abformmassen physikalisch untersucht. Die Ergebnisse sind der nachfolgenden Tabelle zu entnehmen. Es bleibt festzuhalten, dass Polyether-Abformmassen (sowohl ringöffnend über eine Aziridinogruppe als auch durch Kondensationsreaktion vernetzende) gute Gebrauchseigenschaften sowohl bei Bestrahlung bei 20 kGy als auch bei 40 kGy behalten. Das trifft auch auf kondensationsvernetzende Silicon-Abformmaterialien zu.

Bei den additionsvernetzenden Silicon-Abformmassen treten durch "Vorvernetzung" Viskositätserhöhungen und einhergehend Vernetzungskinetikbeschleunigungen auf, die unerwünscht sind, auch wenn die bei 20 kGy behandelte Probe von Provil Novo Medium C.D. 2 trotz der Veränderungen noch brauchbare Abformmasse ergibt (mit Gummieigenschaften nach der Abformung, die keine wesentlichen Änderungen gezeigt haben).

In dem vorgenannten additionsvernetzenden Silicon-Abformmaterial "Provil Novo Medium C.D. 2" liegen in der Basispaste SiH-Vernetzer neben Polydimethylsiloxanen mit endständigen Vinylgruppen vor. Trennung von SiH-Vernetzer und vinylgruppenhaltigen Siloxanpolymeren führt insbesondere bei Verwendung von Diphenylsiloxaneinheiten enthaltenden Polymeren zu geringeren Viskositätsveränderungen, die aber immer noch mit Änderungen der Vernetzungskinetik einhergehen und daher keine generelle Problemlösung darstellen. Strahlendosen von 40 kGy führen bei additionsvernetzenden Silicon-Abformmassen grundsätzlich zu einer Polymerisation und damit bedingt einem viskoelastischen Verhalten der Pasten, die eine Verwendung unmöglich machen. Es lässt sich also erkennen, dass additionsvernetzende Silicon-Abformmaterialien sich bei Sterilisation durch energiereiche Bestrahlung empfindlicher verhalten als andere Abformmassen (ohne dass die grundsätzliche Verwendbarkeit bestrahlter additionsvernetzender Silicon-Abformmaterialien in Frage zu stellen ist) und dass hier eine obere Grenze für die materialkundlich tolerierbare Strahlendosis erkennbar wird.

### Ergebnisse der γ-Bestrahlungsversuche bei verschiedenen Strahlendosen mit verschiedenen Abformmassetypen

| **Material** | | **Polyether-Abformmaterial** | | | | | | **Silicon-Abformmaterialien** | | | | | | | | |
|---|---|---|---|---|---|---|---|---|---|---|---|---|---|---|---|---|
| | | ESPE Impregum Garant L Duo Soft, B: 007/C: 007 | | | B: URH 0246-4/ C: URH 0196-2 | | | Xantopren Comfort Medium, Lot:160182 | | | Provil Novo Medium* C.D. 2, Lot 150352 | | BER 083-2/BER 083-3* | | BER 083/BER 083-1* | |
| Vernetzungsmechanismus zum Elstomer | | über Aziridinogruppe | | | kondensationsvernetzend | | | kondensationsvernetzend | | | addidonsvernetzend | | additionsvernetzend | | additionsvernetzend | |
| Packmittel | | Doppelkammerkartusche, 2:1 | | | Doppelkammerkartusche, 4:1 | | | Doppelkammerkartusche, 4:1 | | | Doppelkammerkartusche, 1:1 | | Doppelkammerkartusche, 4:1 | | Doppelkammerkartusche, 4:1 | |
| Dosis | | "O-Pro be" | 20 kGy | 40 kGy | "O-Pro be" | 20 kGy | 40 kGy | "O-Pro be" | 20 kGy | 40 kGy | "O-Probe" | 20 kGy | "O-Pro be" | 20 kGy | "O-Pro be" | 20 kGy |
| Viskosität Basis | [Pas] | 84 | 77 | 78 | 43 | 42 | 43 | 25 | 27 | 31 | 60 | 164 | 45 | 26 | 43 | 22 |
| Viskosität Katalysator | [Pas] | 249 | 162 | 146 | 35 | 31 | 34 | 3 | 3 | 3 | 58 | 102 | 88 | 127 | 71 | 149 |
| Verarbeitungszeit nach OSC-Messung | [min] | 2,45 | 2,40 | 2,30 | 1,60 | 1,85 | 1,70 | 2,40 | 2,40 | 2,50 | 2,20 | 1,00 | 3,10 | 1,65 | 2,20 | nicht messbar |
| Abbindezeit nach OSC-Messung | [min] | 3,50 | 3,40 | 3,30 | 3,25 | 4,05 | 3,60 | 3,95 | 4,03 | 4,23 | 3,00 | 2,30 | 4,20 | 3,25 | 3,10 | 2,55 |
| Rückstellung nach Verformung | [%] | 97,7 | 97,5 | 97,2 | 98,2 | 98,5 | 98,0 | 96,1 | 96,5 | 96,6 | 99,7 | 99,8 | 98,4 | 98,2 | 98,2 | 99,1 |
| Shore A-Härte nach 1 h | | - | 52 | 52 | 40 | 39 | 38 | 39 | 40 | 42 | 56 | 55 | | | 62 | 63 |

| | | | | | | | | | | | | | | | | |
|---|---|---|---|---|---|---|---|---|---|---|---|---|---|---|---|---|
| *40 kGy: Basis und Katalysator viskoelastisch, nicht mischbar BER 083: Basispaste unter anderem mit SiH-Vernetzer, ohne Vinylöl; BER 083-1: Katalysatorpaste unter anderem mit Polydimethylsiloxanen mit endständigen Vinylgruppen und einer Viskosität von 1.000 mPas beziehungsweise 65.000 mPas BER 083-2: Basispaste unter anderem mit SiH-Vernetzer, ohne Vinylöl; BER 083-3: Katalysatorpaste unter anderem mit Vinylölen der allgemeinen Struktur H₂C = CH-Si(CH₃)₂-O-[Si(CH₃)₂-O]ₘ-[Si(C₆H₃)₂-O]ₙSi(CH₃)₂-CH = CH₂ mit jeweils 3,0 - 3,5 Mol% Diphenylsiloxaneinheiten und einer Viskosität von 1.000 beziehungsweise 60.000 mPas. | | | | | | | | | | | | | | | | |

### Beispiel 3:

In dieser Versuchsserie wurden Veränderungen von additionsvernetzenden zweikomponentigen Silicon-Abformmassen bezüglich der Eigenschaftsveränderung bei Gammastrahlensterilisation bei 25 kGy gegenüber nicht sterilisierten Mustern getestet.

Dabei stellt die Variante BER 091-12/091-13 eine Variante dar, die bezüglich der Vinylgruppen enthaltenden Polymere aus zwei Polydimethylsiloxanen mit jeweils zwei endständigen Vinylgruppen aufgebaut ist. Diese Polymere weisen Viskositäten von ca. 1000 mPas beziehungsweise 65000 mPas auf.

Variante BER 091-4/091-5 enthält stattdessen entsprechende Vinylgruppen enthaltende Polymere, die neben den Dimethylsiloxaneinheiten auch einen Gehalt von 3,0 - 3,5 Mol-% Diphenylsiloxaneinheiten aufweisen.

In BER 091-10/091-11 wird ein entsprechendes Polymer mit circa 10 Mol-% Phenylmethylsiloxaneinheiten der Firma Wacker Chemie GmbH eingesetzt.

Die entsprechenden Daten zeigt die beiliegende Tabelle.

Erwartungsgemäß zeigt sich bei γ-Strahlensterilisation von BER 091-12/091-13 wiederum ein Viskositätsanstieg mit einhergehender Vernetzungskinetikbeschleunigung, der mit dem Diphenysiloxangruppen enthaltenden Polymer, das in BER 091-4/091-5 Verwendung findet, deutlich geringer ausfällt.

Die geringsten Veränderungen lassen sich mit dem in BER 091-10/091-11 verwendeten Polymer erzielen. Das heißt, durch die Variation der verwendeten Polymere in den additionsvernetzenden Siliconabformmassen lassen sich die Veränderungen der Konsistenz beziehungsweise der Kinetik gezielt beeinflussen. Auf diese Weise werden die entsprechenden Abformmassen bezüglich ihrer Verwendbarkeit nach Gammastrahlensterilisation optimiert.

### Ergebnisse von verschiedenen additionsvernetzenden Siliconabformmaterialien, sterilisiert mit γ-Strahlen bei 25 kGy (unsterilisiert zum Vergleich):

| **BER** | | **091-4/091-5** | **091-10/091-11** | **091-12/091-13** |
|---|---|---|---|---|
| Viskosität Basis | [Pas] | 230 | 25 | 351 |
| | | (100) | (34) | (72) |
| Viskosität Katalysator | [Pas] | 178 | 86 | 198 |
| | | (154) | (70) | (132) |
| Verarbeitungszeit (OSC) | [min] | 1,70 | 2,25 | 1,30 |
| | | (3,10) | (3,15) | (3,10) |
| Abbindezeit (OSC) | [min] | 3,10 | 3,25 | 2,80 |
| | | (4,20) | (4,15) | (4,35) |
| Rückstellung nach Verformung | [%] | 99,5 | 99,4 | 99,5 |
| | | (99,6) | (99,0) | (99,5) |

| | | | | |
|---|---|---|---|---|
| * zu schnell * zu langsam 091-4: 18,00% PDV 0331 (ABCR); 9,50% PDV 0346 (ABCR); 16% SiH-Vernetzer; 55,5% Füllstoff, sonstige Stell- und Strukturmittel 091-5: 7,50% PDV 0346 (ABCR); 36,00% PDV 0331 (ABCR); 55,4% Füllstoff; 80 ppm Pt als Pt-dvds-Katalysator (bezogen auf die Kat.-Paste); sonstige Stell- und Strukturmittel 091-10:28,0% SLM 435064/10; 16% SiH-Vernetzer; 54,7% Füllstoff; sonstige Stell- und Strukturmittel 091-11:42,0% SLM 435064/10; 56,4% Füllstoff-, 80 ppm Pt als Pt-dvds-Katalysator (bezogen auf die Kat.-Paste); sonstige Stell- und Strukturmittel 091-12:18,0% Silopren U1; 9,50% Silopren U65; 16% SiH-Vernetzer; 55,5% Füllstoff; sonstige Stell- und Strukturmittel 091-13:36,0% Silopren U1; 7,00% Silopren U65; 55,4% Füllstoff; 80 ppm Pt als Pt-dvds-Katalysator (bezogen auf die Kat.-Paste); sonstige Stell- und Strukturmittel Silopren U1, Silopren U65: Handelsprodukte der Firma GE Bayer Silicones; Polydimethylsiloxan mit endständigen Vinylgruppen an beiden Kettenenden dvds: 1,3-Divinyldisiloxan PDV 0331 (ABCR): Viskosität 1000 mPas; MG: 27.000; 3,0 - 3,5 mol-% Diphenylsiloxane; mit Divinylendgruppen PDV 0346 (ABCR): Viskosität 60.000 mPas; MG: 78.000; 3,0 - 3,5 mol% Diphenylsiloxane; mit Divinylendgruppen SLM 435064 (Wacker): Phenylmethylsiloxaneinheiten: Circa 10 mol-%; mit Divinylendgruppen; Viskosität 600 mPas | | | | |

### Beispiel 4:

Die in nachfolgender Tabelle zusammengestellten Ergebnisse der Keimzahlbestimmungen zeigt, dass nach Gammastrahlensterilisation keine vermehrungsfähigen Mikroorganismen mehr nachweisbar waren.

### Ergebnisse der Keimzahlbestimmungen gemäß DIN EN 1174-1, -2, ISO 11737-1:

| **Material** | **Unbehandelte Probe** | | **Mit Gammastrahlen behandelt** | |
|---|---|---|---|---|
| | Keimzahl Bakterien [KBE/g] | Keimzahl Pilze, Hefen [KBE/g] | Dosis | Ergebnis der Sterilitätsprüfung |
| C-Silicon Xantopren Comfort Medium | < 10 | < 10 | 25 kGy | Es wurden keine vermehrungsfähigen Mikroorganismen nachgewiesen |
| Polyether-Abformmassen Aziridinopolyether Impregum Garant L Duo | Soft < 10 | < 10 | 20 kGy | Es wurden keine vermehrungsfähigen Mikroorganismen nachgewiesen |
| kondensationsvernetzbare Polyether-Abformmasse (URH 0246-4/URH 0196-2) | < 10 | < 10 | 20 kGy | Es wurden keine vermehrungsfähigen Mikroorganismen nachgewiesen |
| Additionsvernetzende Silicon-Abformmasse BER 083-2/083-3 | < 10 | < 10 | 20 kGy | Es wurden keine vermehrungsfähigen Mikroorganismen nachgewiesen |

### Beispiel 5:

Elektronenstrahlsterilisationsversuche an verschiedenen Abformmassetypen:

Die beiden nachfolgenden Tabellen zeigen vergleichende Daten von verschiedenen Abformmassetypen in unsterilisiertem Zustand und nach einer Elektronenstrahlsterilisation mit einer Dosis von jeweils 25 kGy. Die untersuchten elektronenstrahlbehandelten Proben wiesen keine vermehrungsfähigen Mikroorganismen auf.

In Analogie zu den Ergebnissen bei Gammastrahlenbehandlung zeigen kondensationsvernetzende Silicon-Abformmassen beziehungsweise Polyether-Abformmassen nur geringe Beeinflussung der physikalischen Eigenschaften.

In einer weiteren Tabelle finden sich Beispiele zu additionsvernetzenden Silicon-Abformmassen, wobei die Variante BER 091-12/091-13 Polydimethylsiloxane mit jeweils endständigen Vinylgruppen als vinylgruppenenthaltende Öle enthalten. Der Viskositätsanstieg ist deutlich, ebenso die damit einhergehende Kinetikbeschleunigung, die aber auch nach der Strahlensterilisation noch zu gut handhabbaren Silicon-Abformmaterialien führt. Noch deutlich geringere Veränderungen sind mit PDV 0331/0346 beziehungsweise SLM 435064 in BER 091-4/091-5 beziehungsweise BER 091-10/091-11 erzielbar.

| **Materialtyp** | | **Silicon-Abformmateriallen, additionsvernetzend** | | | | | |
|---|---|---|---|---|---|---|---|
| **Packmittel** | | **Doppelkammerkartuschen 1 :** 1 | | | | | |
| **BER** | | **091-4/091-5** | | **091-10/091-11** | | **091-12/091-13** | |
| | | vor der Sterilisierung | elektro nenstrahlsterilisiert, 25 kGy | vor der Sterilisierung | elektronenstrahlsterilisiert, 25 kGy | vor der Sterilisierung | elektronenstrahlsterilisiert, 25 kGy |
| Viskosität Basis | [Pas] | 100 | 132 | 34 | 29 | 72 | 132 |
| Viskosität Katalysator | [Pas] | 154 | 183 | 70 | 80 | 132 | 152 |
| Verarbeitungszeit (Handhabungstest) | [min] | 4,68 | 3,85 | 5,45 | 5,23 | 5,40 | 3,45 |
| Verarbeitungszeit (nach OSC-Methode) | [min] | 3,10 | 2,50 | 3,15 | 2,80 | 3,10 | 2,10 |
| Abbindezeit (nach OSC-Methode) | [min] | 4,20 | 4,05 | 4,15 | 3,90 | 4,35 | 3,55 |
| Rückstellung nach Verformung | [%] | 99,6 | 99,5 | 99,0 | 98,2 | 99,5 | 99,7 |
| Keimzahl Bakterien | [KBE/g] | - | - | < 10 | * | < 10 | * |
| Keimzahl Pilze/Hefen | [KBE/g] | - | - | < 10 | * | < 10 | * |

| | | | | | | | |
|---|---|---|---|---|---|---|---|
| BER 091-12/-13: mit Silopren U 1/U 65, das heißt, Polydimethylsiloxan mit endständigen Vinylgruppen der Viskositäten 1 Pas beziehungsweise 65 Pas BER 091-10/-11: mit SLM 435064 BER 091-4/-5: mit PDV 0331/PDV 0346 (ABCR), das heißt, Polydimethylsiloxanen mit endständigen Vinylgruppen mit einem Gehalt an 3,0 - 3,5 Mol-% Diphenylsiloxaneinheiten in der Kette mit Viskositäten von 1 Pas beziehungsweise 60 Pas * vermehrungsfähige Mikroorganismen wurden nicht nachgewiesen | | | | | | | |

| **Materialtyp** | | **Silicon-Abformmaterial, kondensationsvernetzend** | | **Polyether-Abformmaterial, kondensationsvernetzend** | | | |
|---|---|---|---|---|---|---|---|
| **Material** | | **Xantopren Comfort Medium** | | **URH 265-1/URH 234-2** | | **URH 260-1/URH 258-2** | |
| | | vor der Sterilisierung | nach Elektronenstrahlsterilisation | vor der Sterilisierung | nach Elektronenstrahlsterilisation | vor der Sterilisierung | nach Elektronenstrahlsterilisation |
| Dosis | | - | 25 kGy* | - | 25 kGy* | - | 25 kGy |
| Packmittel | | Doppelkammerkartusche, 4 : 1 | | Doppelkammerkartusche, 4 : 1 | | Doppelkammerkartusche, 2 : 1 | |
| Viskosität Basis | [Pas] | 16 | 21 | 116 | 104 | 112 | 114 |
| Viskosität Katalysator[ | Pas] | - | 3 | 75 | 67 | 83 | 71 |
| Verarbeitungszeit (OSC-Methode) | [min] | 2,25 | 2,15 | 1,57 | 1,93 | 1,50 | 1,70 |
| Abbindezeit (OSC-Methode) | [min] | 3,35 | 4,00 | 2,70 | 3,53 | 2,40 | 2,50 |
| Rückstellung nach Verformung | [%] | 98,1 | 98,2 | 99,0 | 98,9 | 98,7 | 98,4 |
| Klebfreitest | [min] | - | - | - | - | - | - |

| | | | | | | | |
|---|---|---|---|---|---|---|---|
| * vermehrungsfähige Mikroorganismen wurden nicht nachgewiesen | | | | | | | |

## Patentansprüche

1. Verfahren zur Keimreduktion von Komponenten zweikomponentiger, zu einem elastomeren Material vernetzenden Abformmaterialien vor der Vernetzung, wobei die Abformmaterialien aus der Gruppe ausgewählt sind, die aus (i) kondensationsvernetzbaren Silicon-Abformmassen, (ii) über (Meth)acrylatgruppen vernetzbaren Silicon-Abformmassen, (iii) additionsvernetzbaren Polyetherabformmassen, (iv) kondensationsvernetzbaren Polyetherabformmassen, (v) über Ringöffnung vernetzbaren Polyetherabformmassen und (vi) über (Meth)acrylatgruppen vernetzbaren Polyetherabformmassen besteht, **dadurch gekennzeichnet, dass**
die Komponenten in einem Primärpackmittel vorliegen und darin einer Strahlensterilisation unterzogen werden, wobei die Strahlung aus der Gruppe ausgewählt wird, die aus Gammastrahlung und Elektronenstrahlung besteht, und die Strahlendosis im Bereich von 20 kGy bis 50 kGy liegt.

2. Verfahren zur Keimreduktion von Komponenten zweikomponentiger, zu einem elastomeren Material vernetzenden Abformmaterialien vor der Vernetzung, wobei die Abformmaterialien aus der Gruppe ausgewählt sind, die aus additionsvernetzbaren Silicon-Abformmassen besteht, die in der Formulierung vinylgruppenhaltige Polysiloxane mit zumindest teilweise vorhandenen Diphenylsiloxan- und/oder Phenylmethylsiloxan-Struktureinheiten aufweist, **dadurch gekennzeichnet, dass**
die Komponenten in einem Primärpackmittel vorliegen und darin einer Strahlensterilisation unterzogen werden, wobei die Strahlung aus der Gruppe ausgewählt wird, die aus Gammastrahlung und Elektronenstrahlung besteht, und die Strahlendosis im Bereich von 20 kGy bis 30 kGy liegt.

3. Verfahren nach Anspruch 2, **dadurch gekennzeichnet, dass** Polymere verwendet werden, die mindestens 3 Mol-%, vorzugsweise mindestens 10 Mol-% Diphenylsiloxan und/oder Phenylmethylsiloxan-Einheiten aufweisen.

4. Verfahren nach einem der Ansprüche 1 bis 3, **dadurch gekennzeichnet, dass** das Primärpackmittel mit dem Abformmaterial gleichzeitig mit Zubehör zum Mischen oder zur Applikation des Abformmaterials behandelt wird.

5. Verfahren nach Anspruch 4, **dadurch gekennzeichnet, dass** als Primärpackmittel eine Doppelkammerkartusche und als Zubehör eine Mischdüse verwendet werden.

6. Verwendung des Verfahrens nach einem der Ansprüche 1 bis 5 für im medizinischen Bereich verwendete Abformmassen.

7. Verwendung nach Anspruch 6 für Abformmassen im Dentalbereich, in der Orthopädie, in der Otoplastik, in der Epithetik, in der Defektchirurgie, der Veterinärmedizin, im Bereich der Abformung in der HNO-Heilkunde oder zur Abformung von Hautpartien.

## Claims

1. Method for germ reduction, prior to cross-linking, of components of two-component impression materials that cross-link to form an elastomeric material, whereby the impression materials are selected from the group consisting of (i) silicone impression masses that can be cross-linked through condensation, (ii) silicone impression masses that can be cross-linked through (meth)acrylate groups, (iii) polyether impression masses that can be cross-linked through addition, (iv) polyether impression masses that can be cross-linked through condensation, (v) polyether impression masses that can be cross-linked through ring opening, and (vi) polyether impression masses that can be cross-linked through (meth)acrylate groups,
**characterised in that**
the components are present in primary packaging means and are subjected to a radiation sterilisation therein, whereby the radiation is selected from the group consisting of gamma radiation and electron radiation, and the radiation dose is in the range from 20 kGy to 50 kGy.

2. Method for germ reduction, prior to cross-linking, of components of two-component impression materials that cross-link to form an elastomeric material, whereby the impression materials are selected from the group consisting of silicone impression masses that can be cross-linked through addition, which includes in the formulation vinyl group-containing polysiloxanes with diphenylsiloxane and/or phenylmethylsiloxane structural moieties being present at least in part, **characterised in that**
the components are present in primary packaging means and are subjected to a radiation sterilisation while being present therein, whereby the radiation is selected from the group consisting of gamma radiation and electron radiation, and the radiation dose is in the range from 20 kGy to 30 kGy.

3. Method according to claim 2, **characterised in that** polymers including at least 3 mol-%, preferably at least 10 mol-%, diphenylsiloxane and/or phenylmethylsiloxane moieties are used.

4. Method according to any one of the claims 1 to 3, **characterised in that** the primary packaging means containing the impression material and accessories for mixing or for application of the impression material are treated simultaneously.

5. Method according to claim 4, **characterised in that** a double-chamber cartridge is used as primary packaging means and a mixing nozzle is used as accessories.

6. Use of the method according to any one of the claims 1 to 5 for impression masses that are used in the field of medicine.

7. Use according to claim 6 for impression masses in dentistry, in orthopaedics, in otoplasty, in epithetics, in defect surgery, in veterinary medicine, in impression-taking applications in ENT or for impression-taking of regions of the skin.

## Revendications

1. Procédé de réduction bactérienne de composants de matériaux de moulage bi-composants, se réticulant en un matériau élastomère avant la réticulation, dans lequel les matériaux de moulage sont sélectionnés parmi le groupe qui se compose de (i) masses de moulage en silicone à réticulation par condensation, (ii) masses de moulage en silicone à réticulation par le biais de groupes (méth)acrylate, (iii) masses de moulage de polyéther à réticulation par addition, (iv) masses de moulage de polyéther à réticulation par condensation, (v) masses de moulage de polyéther à réticulation par ouverture de cycle et (vi) masses de moulage de polyéther à réticulation par le biais de groupes (méth)acrylate, **caractérisé en ce que**
les composants sont présents dans un emballage primaire et y sont soumis à une stérilisation par rayonnement, dans lequel le rayonnement est sélectionné parmi le groupe qui se compose d'un rayonnement gamma et d'un faisceau électronique, et la dose de rayonnement se situe dans la région de 20 kGy à 50 kGy.

2. Procédé de réduction bactérienne de composants de matériaux de moulage bi-composants, se réticulant en un matériau élastomère avant la réticulation, dans lequel les matériaux de moulage sont sélectionnés parmi le groupe qui se compose de masses de moulage en silicone à réticulation par addition, qui présente dans la formulation des polysiloxanes contenant des groupes vinyle avec des motifs structurels de diphénylsiloxane et/ou phénylméthylsiloxane présents au moins en partie, **caractérisé en ce que**
les composants sont présents dans un emballage primaire et y sont soumis à une stérilisation par rayonnement, dans lequel le rayonnement est sélectionné parmi le groupe qui se compose d'un rayonnement gamma et d'un faisceau électronique, et la dose de rayonnement se situe dans la région de 20 kGy à 30 kGy.

3. Procédé selon la revendication 2, **caractérisé en ce que** des polymères qui présentent au moins 3 % en mole, de préférence au moins 10 % en mole de motifs diphénylsiloxane et/ou phénylméthylsiloxane, sont utilisés.

4. Procédé selon l'une quelconque des revendications 1 à 3, **caractérisé en ce que** l'emballage primaire avec le matériau de moulage est manipulé en même temps avec des accessoires pour le mélange ou pour l'application du matériau de moulage.

5. Procédé selon la revendication 4, **caractérisé en ce qu'**une cartouche à double chambre est utilisée comme emballage primaire et une buse mixte est utilisée comme accessoire.

6. Utilisation du procédé selon l'une quelconque des revendications 1 à 5 pour des masses de moulage utilisées dans le domaine médical.

7. Utilisation selon la revendication 6 pour des masses de moulage dans le domaine dentaire, en orthopédie, en otoplastique, en épithétique, dans la chirurgie réparatrice, la médecine vétérinaire, dans le domaine du moulage en oto-rhinolaryngologie ou pour le moulage de parties corporelles.
